(19) Europäisches Patentamt
European Patent Office
Office européen des brevets

(11) **EP 2 714 104 B1**

(12) **EUROPÄISCHE PATENTSCHRIFT**

(45) Veröffentlichungstag und Bekanntmachung des Hinweises auf die Patenterteilung:
**29.07.2015   Patentblatt 2015/31**

(21) Anmeldenummer: **12723208.0**

(22) Anmeldetag: **24.05.2012**

(51) Int Cl.:
*A61L 15/22* (2006.01)     *A61L 15/60* (2006.01)
*B01F 7/04* (2006.01)     *B01F 11/00* (2006.01)
*C08L 33/02* (2006.01)     *B01F 15/06* (2006.01)

(86) Internationale Anmeldenummer:
**PCT/EP2012/059777**

(87) Internationale Veröffentlichungsnummer:
**WO 2012/160174 (29.11.2012 Gazette 2012/48)**

(54) **VERFAHREN ZUR HERSTELLUNG WASSERABSORBIERENDER POLYMERPARTIKEL**

METHOD FOR PRODUCING WATER-ABSORBING POLYMER PARTICLES

PROCÉDÉ DE PRODUCTION DE PARTICULES POLYMÈRES ABSORBANT L'EAU

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.05.2011   EP 11167609**

(43) Veröffentlichungstag der Anmeldung:
**09.04.2014   Patentblatt 2014/15**

(73) Patentinhaber: **BASF SE**
**67056 Ludwigshafen (DE)**

(72) Erfinder:
• **WEISMANTEL, Matthias**
**63637 Jossgrund (DE)**
• **RIEGEL, Ulrich**
**66849 Landstuhl (DE)**
• **BRAUN, Markus**
**69118 Heidelberg (DE)**
• **WENDKER, Martin**
**67549 Worms (DE)**
• **GIEGER, Thomas**
**67069 Ludwigshafen (DE)**

(56) Entgegenhaltungen:
**WO-A1-2004/069404     WO-A2-2004/069915
DE-A1- 19 955 861**

EP 2 714 104 B1

**Beschreibung**

Die vorliegende Erfindung betrifft ein Verfahren zur Herstellung wasserabsorbierender Polymerpartikel durch Polymerisation einer wässrigen Monomerlösung in einem Kneter mit mindestens zwei achsparallel rotierenden Wellen, wobei die Reaktionsmischung in axialer Richtung transportiert und der Bereich am Anfang des Kneters begleitbeheizt wird.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm$^2$ (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49,2 g/cm$^2$ (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm$^2$ (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die Herstellung wasserabsorbierender Polymerpartikel in einem Kneter mit mindestens zwei achsparallel rotierenden Wellen wird beispielsweise in WO 01/038402 A1, WO 03/022896 A1 und WO 2006/034806 A1 beschrieben.

Gemäß WO 01/038402 A1 wird die Reaktionswärme zu mindestens 25% durch Verdampfung von Wasser abgeführt.

WO 03/022896 A1 lehrt, dass zumindest ein Teil des in den Kneter dosierten Wassers als Wasserdampf zugeführt wird.

Die WO 2006/034806 A1 betrifft weitere Aspekte der Polymerisation wie den Füllgrad des Kneters, den Inhibitorgehalt der Monomerlösung, die Temperatur in der Reaktionszone und die Rückvermischung während der Polymerisation.

Aufgabe der vorliegenden Erfindung war die Bereitstellung eines verbesserten Verfahrens zur Herstellung wasserabsorbierender Polymerpartikel, insbesondere von wasserabsorbierenden Polymerpartikeln mit hoher Zentrifugenretentionskapazität (CRC) und wenig extrahierbaren Anteilen (Extrahierbaren).

Gelöst wurde die Aufgabe durch ein Verfahren zur Herstellung wasserabsorbierender Polymerpartikel durch Polymerisation einer Monomerlösung oder -suspension, enthaltend

a) ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

in einem Kneter mit einem Reaktionsvolumen von mindestens 0,1 m$^3$ und mindestens zwei achsparallel rotierenden Wellen, wobei sich auf den Wellen mehrere Knet- und Transportelemente befinden, die eine Förderung der am Anfang des Kneters zugegebenen Reaktionsmischung in axialer Richtung zum Ende des Kneters bewirken, dadurch gekennzeichnet, dass die Monomerlösung von 0,01 bis 0,2 Gew.-% Initiator c), bezogen auf unneutralisiertes Monomer a), enthält, die Kneteraußenwand im Bereich des Zulaufs begleitbeheizt wird und die Temperatur der Begleitheizung mindestens 110°C beträgt.

Die Monomerlösung enthält vorzugsweise von 0,02 bis 0,18 Gew.-%, besonders bevorzugt von 0,04 bis 0,16 Gew.-%, ganz besonders bevorzugt von 0,05 bis 0,15 Gew.-%, Initiator c), jeweils bezogen auf unneutralisiertes Monomer a).

In eine besonders bevorzugten Ausführungsform der vorliegenden Erfindung ist der Initiator c) zu vorzugsweise mindestens 50 Gew.-%, besonders bevorzugt zu mindestens 70 Gew.-%, ganz besonders bevorzugt zu mindestens 80 Gew.-%, ein Peroxid. In einer ganz besonders bevorzugten Ausführungsform der vorliegenden Erfindung ist der Initiator c) zu vorzugsweise mindestens 50 Gew.-%, besonders bevorzugt zu mindestens 70 Gew.-%, ganz besonders bevorzugt zu mindestens 80 Gew.-%, Natriumperoxodisulfat.

Die Temperatur der Begleitheizung beträgt vorzugsweise mindestens 115°C, besonders bevorzugt mindestens 118°C, ganz besonders bevorzugt mindestens 120°C.

Durch die Begleitheizung im vorderen Bereich des Kneters zerfallen die meisten Initiatoren möglicherweise bereits in der Nähe des Zulaufs. Bei einer damit verbundenen entsprechend längeren Verweilzeit der frühzeitig gebildeten Startradikale kann bereits mit weniger Initiator ein hoher Monomerumsatz erreicht werden. Durch weniger Initiator wiederum wird das Verhältnis von Zentrifugenretentionskapazität (CRC) und extrahierbaren Anteilen (Extrahierbaren) günstig beeinflusst. Peroxide wie Natriumperoxodisulfat zerfallen thermisch und können daher besonders leicht durch entsprechende Temperaturkontrolle beeinflusst werden.

Eine ebenfalls mögliche Verlängerung der Verweilzeit im Kneter würde zwar auch zu einem hohen Monomerumsatz führen, aber gleichzeitig in unerwünschter Weise die Kapazität senken.

Diese Wirkung hängt möglicherweise auch vom Reaktions- bzw. Reaktorvolumen ab. Das Reaktorvolumen beträgt daher vorzugsweise mindestens 0,5 m$^3$, besonders bevorzugt mindestens 1 m$^3$, ganz besonders bevorzugt mindestens 2 m$^3$, wobei das Reaktorvolumen das Füllvolumen des Reaktors ist.

Der vordere Bereich der Kneteraußenwand umfasst vorzugsweise die vorderen 50%, besonders bevorzugt die vorderen 70%, ganz besonders bevorzugt die vorderen 90%, der Kneteraußenwand, wobei die Prozentangaben die Längenprozente des Kneters beginnend mit der Seite des Zulaufs einschließlich der Stirnwand bedeuten.

Es ist wichtig, dass insbesondere der vordere Bereich des Kneters entsprechend begleitbeheizt wird. Dadurch werden die Reaktionstemperaturen bereits frühzeitig angehoben und viele Startradikale gebildet. Es kann auch die gesamte Außenwand begleitbeheizt werden.

Die Reaktoraußenwand wird vorzugsweise mittels eines Doppelmantels begleitbeheizt. Als Wärmeträger sind Warmwasser und Heizdampf geeignet. Hierbei ist Heizdampf bevorzugt, da die Temperatur des Heizdampfes sehr einfach über den Druck eingestellt werden kann. Dies kann beispielsweise durch Entspannung von höher gespanntem Wasserdampf mit anschließender Sättigung des so erzeugten überhitzten Wasserdampfes erfolgen.

Der Heizdampf weist einen Druck von vorzugsweise 1,4 bis 16 bar, besonders bevorzugt von 1,8 bis 11 bar, ganz besonders bevorzugt von 2 bis 4 bar, auf.

Die erfindungsgemäße Begleitheizung ermöglicht die Polymerisation bei deutlich höheren Temperaturen. Die Begleitheizung wird dabei so eingestellt, dass die maximale Temperatur der Reaktionsmischung vorzugsweise mindestens 105°C, besonders bevorzugt mindestens 108°C, ganz besonders bevorzugt mindestens 110°C, beträgt. Überraschenderweise führen die hohen Reaktionstemperaturen zu keiner messbaren Verschlechterung der Produkteigenschaften.

Die im erfindungsgemäßen Verfahren einsetzbaren Kneter weisen mindestens zwei achsparallel rotierende Wellen auf, wobei sich auf den Wellen üblicherweise mehrere Knet- und Transportelemente befinden.

Im erfindungsgemäßen Verfahren einsetzbare Kneter sind beispielsweise von der List AG (Arisdorf; Schweiz) erhältlich und in der CH 664 704 A5, EP 0 517 068 A1, WO 97/12666 A1, DE 21 23 956 A1, EP 0 603 525 A1, DE 195 36 944 A1 und DE 41 18 884 A1 beschrieben.

Solche Kneter mit mindestens zwei Wellen erzielen durch die Anordnung der Knet- und Transportelemente eine hohe Selbstreinigung, die für eine kontinuierliche Polymerisation eine wichtige Anforderung ist. Vorzugsweise rotieren die beiden Wellen gegenläufig zueinander.

Auf der Rührwelle sind die Scheibensegmente propellerartig angeordnet. Als Knet- und Transportelemente sind beispielsweise wandgängige Mischbarren sowie L- oder U-förmig ausgeformte Aufsätze geeignet.

Im Folgenden wird die Herstellung der wasserabsorbierenden Polymerpartikel näher erläutert:

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 02/055469 A1, der WO 03/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 03/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,6 Gew.-%, jeweils bezogen auf unneutralisiertes Monomer a). Mit steigendem Vernetzergehalt sinkt die Zentrifugenretentionskapazität (CRC) und die Absorption unter einem Druck von 21,0 g/cm$^2$ durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Zur Verbesserung der Trocknungseigenschaften kann das erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur $T_g$ auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 $\mu$m, besonders bevorzugt von 250 bis 600 $\mu$m, ganz besonders von 300 bis 500 $\mu$m. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 $\mu$m beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Bei der Polymerisation in einem Knetreaktor werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 $\mu$m, beträgt vorzugsweise mindestens 90 Gew.-%, besonders bevorzugt mindestens 95 Gew.-%, ganz besonders bevorzugt mindestens 98 Gew.-%.

Der Anteil an Partikeln mit einer Partikelgröße von höchstens 600 $\mu$m, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 03/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%, jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA empfohlenen Testmethode Nr. WSP 230.2-05 "Mass Loss Upon Heating" bestimmt wird.

Die gemäß dem erfindungsgemäßen Verfahren hergestellten wasserabsorbierenden Polymerpartikel haben einen Anteil an Partikeln mit einer Partikelgröße von 300 bis 600 µm von vorzugsweise mindestens 30 Gew.-%, besonders bevorzugt mindestens 50 Gew.-%, ganz besonders bevorzugt mindestens 70 Gew.-%.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die gemäß dem erfindungsgemäßen Verfahren hergestellten wasserabsorbierenden Polymerpartikel weisen eine Absorption unter einem Druck von 49,2 $g/cm^2$ von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 $g/cm^2$ der wasserabsorbierenden Polymerpartikel beträgt üblicherweise weniger als 35 g/g. Die Absorption unter einem Druck von 49,2 $g/cm^2$ wird analog der von der EDANA empfohlenen Testmethode Nr. WSP 242.2-05 "Absorption Under Pressure, Gravimetric Determination" bestimmt, wobei statt eines Drucks von 21,0 $g/cm^2$ ein Druck von 49,2 $g/cm^2$ eingestellt wird.

Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, U.S.A., www.inda.org). Diese Veröffentlichung ist sowohl von EDANA als auch von INDA erhältlich.

Die Messungen sollten, wenn nicht anders angegeben, bei einer Umgebungstemperatur von 23 $\pm$ 2 °C und einer relativen Luftfeuchte von 50 $\pm$ 10 % durchgeführt werden. Die wasserabsorbierenden Polymerpartikel werden vor der Messung gut durchmischt.

Quellgeschwindigkeit (Free Swell Rate)

Zur Bestimmung der Quellgeschwindigkeit (FSR) werden 1,00 g (= W1) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W2). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Quellgeschwindigkeit (FSR) wie folgt:

$$\text{FSR [g/g s]} = W2/(W1 x t)$$

Wenn der Feuchtegehalt der wasserabsorbierenden Polymerpartikel jedoch mehr als 3 Gew.-% beträgt, so ist das Gewicht W1 um diesen Feuchtegehalt zu korrigieren.

Flüssigkeitsweiterleitung (Saline Flow Conductivity)

Die Flüssigkeitsweiterleitung (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Flüssigkeitsweiterleitung (SFC) wird wie folgt berechnet:

$$\text{SFC [cm}^3\text{s/g]} = (Fg(t=0) x L0)/(d x A x WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm$^3$, A die Fläche der Gelschicht in cm$^2$ und WP der hydrostatische Druck über der Gelschicht in dyn/cm$^2$.

pH-Wert

Der pH-Wert der wasserabsorbierenden Polymerpartikel wird gemäß der von der EDANA empfohlenen Testmethode Nr. WSP 200.2-02 "pH of Polyacrylate (PA) Powders" bestimmt.

Restmonomer

Der Gehalt an Restmonomer der wasserabsorbierenden Polymerpartikel wird gemäß der von der EDANA empfohlenen Testmethode WSP Nr. 210.2-02 "Residual Monomers" bestimmt.

Feuchtegehalt

Der Feuchtegehalt der wasserabsorbierenden Polymerpartikel wird gemäß der von der EDANA empfohlenen Testmethode Nr. WSP 230.2-02 "Mass Loss Upon Heating" bestimmt.

Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Absorption unter einem Druck von 49,2 g/cm$^2$ (Absorption under Load)

Die Absorption unter einem Druck von 49,2 g/cm$^2$ (AUL0.7psi) wird analog der von der EDANA empfohlenen Testmethode Nr. WSP 242.2-05 "Absorption Under Pressure, Gravimetric Determination" bestimmt, wobei statt eines Drucks von 21,0 g/cm$^2$ (AUL0.3psi) ein Druck von 49,2 g/cm$^2$ (AUL0.7psi) eingestellt wird.

Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

Beispiel 1 (Vergleichsbeispiel)

Durch kontinuierliches Mischen von entionisiertem Wasser, 50gew.-%iger Natronlauge und Acrylsäure wurde eine Acrylsäure/Natriumacrylatlösung hergestellt, so dass der Neutralisationsgrad 71,0 mol-% entsprach. Der Feststoffgehalt der Monomerlösung betrug 42,0 Gew.-%. Als Vernetzer wurde 3-fach ethoxiliertes Glyzerintriacrylat (ca. 85gew.-%ig) verwendet. Die Einsatzmenge betrug 0,383 Gew.-%, bezogen auf unneutralisierte Acrylsäure.

Zur Initiierung der radikalischen Polymerisation wurden 0,0023 Gew.-% Ascorbinsäure, 0,2 Gew.-% Natriumperoxodisulfat und 0,0006 Gew.-% Wasserstoffperoxid, jeweils bezogen auf unneutralisierte Acrylsäure, eingesetzt. Zusätzlich wurden 0,04 Gew.-% Zitronensäure, bezogen auf unneutralisierte Acrylsäure, in die Monomerlosung dosiert.

Zwischen dem Zugabepunkt für Vernetzer und den Zugabestellen für die Wasserstoffperoxid/Natriumperoxodisulfat wurde die Monomerlösung mit Stickstoff inertisiert. Die Zitronensäure wurde unmittelbar vor dem Reaktor und die Ascorbinsäure erst im Reaktor zugesetzt.

Die Reaktion wurde in einem Reaktor vom Typ List ORP 250 Contikneter, (LIST AG; Arisdorf; Schweiz) durchgeführt. Der Durchsatz der Monomerlösung betrug 1200 kg/h. Die Reaktionslösung hatte am Zulauf eine Temperatur von 33°C. Die Temperatur des Wassers im Doppelmantel des Reaktors wurde auf 95°C eingestellt. Die maximale Temperatur der Reaktionsmischung betrug 104°C. Die Verweilzeit der Reaktionsmischung im Reaktor betrug 9 Minuten.

Das erhaltene Polymergel wurde auf einen Bandtrockner aufgegeben. Auf dem Bandtrockner wurde das Polymergel kontinuierlich mit einem Luft/Gasgemisch umströmt und bei 175°C getrocknet. Die Verweilzeit im Bandtrockner betrug 43 Minuten.

Das getrocknete Polymergel wurde gemahlen und auf eine Partikelgrößenfraktion von 200 bis 850 μm abgesiebt. Das so erhaltene Grundpolymer hatte folgende Eigenschaften:

CRC:                    36,3 g/g

(fortgesetzt)

| Extrahierbare: | 13,9 Gew.-% |
|---|---|
| Feuchtegehalt: | 2,1 Gew.-% |
| Restmonomere: | 300 ppm |

In einem Schugi Flexomix® vom Typ: FX 160 (Hosokawa-Micron B.V.; Doetinchem; Niederlande) wurde das Grundpolymer mittels getrennter Zweistoffdüsen mit einer Oberflächennachvernetzungslösung und einer Aluminiumlaktatlösung beschichtet und anschließend direkt in einem NARA-Paddle-Dryer vom Typ NPD 5W8 (GMF Gouda; Waddinxveen; Niederlande) 45 Minuten bei 195°C getrocknet.

Es wurden folgende Mengen in den Schugi Flexomix® dosiert:

| 500 kg/h | Grundpolymer |
|---|---|
| 15,3 kg/h | Oberflächennachvernetzungslösung |
| 3,00 kg/h | 25gew.-%ige wässrige Aluminiumlaktatlösung |

Die Oberflächennachvernetzungslösung enthielt 2.0 Gew.-% N-Hydroxyethyl-2-oxazolidinon, 2,0 Gew.-% 1,3-Propandiol, 35,6 Gew.-% Isopropanol, 60,4 Gew.-% entionisiertes Wasser und 0,03 Gew.-% Sorbitanmonolaurat.

Die oberflächennachvernetzten Polymerpartikel wurden anschließend in einem NARA-Paddle-Cooler vom Typ NPD 3W9 (GMF Gouda; Waddinxveen; Niederlande) auf ca. 60°C abgekühlt, mit 2,06 kg/h Wasser nachbefeuchtet und anschließend noch einmal auf 200 bis 850 $\mu$m abgesiebt.

Die verwendeten oberflächennachvernetzten wasserabsorbierenden Polymerpartikel hatten folgendes Eigenschaftsprofil:

| CRC: | 29,3 g/g |
|---|---|
| AUL0.7psi: | 24,3 g/g |
| SFC: | $41 \times 10^{-7}$ cm$^3$s/g |
| FSR | 0,15 g/gs |
| pH-Wert | 6,1 |
| Extrahierbare: | 9,7 Gew.-% |
| Feuchtegehalt | 0,2 Gew.-% |
| Restmonomer | 350 ppm |

Beispiel 2

Es wurde verfahren wie unter Beispiel 1. Die Temperatur des Reaktordoppelmantels wurde mittels Heizdampfes auf 120°C eingestellt. Die maximale Temperatur der Reaktionsmischung betrug 112°C. Die Menge des Initiators Natriumperoxodisulfat wurde in drei Stufen gesenkt. Die Ergebnisse sind in den Tabellen 1 und 2 zusammengefasst.

Tab. 1: Grundpolymer

| Natriumpersulfat [Gew.-%]*) | 0,2 | 0,184 | 0,164 | 0,147 |
|---|---|---|---|---|
| CRC [g/g] | 36,3 | 36,6 | 36,6 | 36,3 |
| Extrahierbare [Gew.-%] | 13,9 | 13,1 | 13,0 | 12,1 |
| Feuchtegehalt [Gew.-%] | 2,1 | 1,8 | 1,8 | 1,9 |
| Restmonomere [ppm] | 300 | 315 | 365 | 370 |
| *) bezogen auf unneutralisierte Acrylsäure | | | | |

Tab. 2: Endprodukt

| Natriumpersulfat [Gew.-%]*) | 0,2 | 0,184 | 0,164 | 0,147 |
|---|---|---|---|---|
| CRC [g/(g] | 29,3 | 29,7 | 29,2 | 29,6 |

(fortgesetzt)

| | | | | |
|---|---|---|---|---|
| AUL0.7psi [g/(g] | 24,3 | 24,7 | 24,1 | 24,6 |
| SFC [$10^{-7}$ cm$^3$s/g] | 41 | 41 | 41 | 36 |
| FSR [g/gs] | 0,15 | 0,16 | 0,16 | 0,17 |
| pH | 6,1 | 6,0 | 6,0 | 6,0 |
| Extrahierbare [Gew.-%] | 9,7 | 9,3 | 8,8 | 8,6 |
| Feuchtegehalt [Gew.-%] | 0,2 | 0,2 | 0,3 | 0,2 |
| Restmonomere [Gew.-%] | 350 | 375 | 400 | 450 |
| *) bezogen auf unneutralisierte Acrylsäure | | | | |

Die Ergebnisse zeigen, dass sich die Initiatormenge durch die Begleitheizung bei nahezu gleichbleibend niedrigem Gehalt an Restmonomeren deutlich senken lässt. Gleichzeitig verbessert sich das Verhältnis CRC/Extrahierbare zu deutlich günstigeren Werten.

**Patentansprüche**

1.  Verfahren zur Herstellung wasserabsorbierender Polymerpartikel durch Polymerisation einer Monomerlösung oder -suspension, enthaltend

    a) ein ethylenisch ungesättigtes, säuregruppentragendes Monomer, das zumindest teilweise neutralisiert sein kann,
    b) mindestens einen Vernetzer,
    c) mindestens einen Initiator,
    d) optional ein oder mehrere mit den unter a) genannten Monomeren copolymerisierbare ethylenisch ungesättigte Monomere und
    e) optional ein oder mehrere wasserlösliche Polymere,

    in einem Kneter mit einem Reaktionsvolumen von mindestens 0,1 m$^3$ und mindestens zwei achsparallel rotierenden Wellen, wobei sich auf den Wellen mehrere Knet- und Transportelemente befinden, die eine Förderung der am Anfang des Kneters zugegebenen Reaktionsmischung in axialer Richtung zum Ende des Kneters bewirken, **dadurch gekennzeichnet, dass** die Monomerlösung von 0,01 bis 0,2 Gew.-% Initiator c), bezogen auf unneutralisiertes Monomer a), enthält, die Kneteraußenwand im Bereich des Zulaufs begleitbeheizt wird und die Temperatur der Begleitheizung mindestens 110°C beträgt.

2.  Verfahren gemäß Anspruch 1, **dadurch gekennzeichnet, dass** der Initiator c) zu mindestens 50 Gew.-% ein Peroxid ist.

3.  Verfahren gemäß Anspruch 1 oder 2, **dadurch gekennzeichnet, dass** der Initiator c) zu mindestens 50 Gew.-% Natriumperoxodisulfat ist.

4.  Verfahren gemäß einem der Ansprüche 1 bis 3, **dadurch gekennzeichnet, dass** die vorderen 50% der Kneteraußenwand begleitbeheizt werden.

5.  Verfahren gemäß einem der Ansprüche 1 bis 4, **dadurch gekennzeichnet, dass** die gesamte Kneteraußenwand begleitbeheizt wird.

6.  Verfahren gemäß einem der Ansprüche 1 bis 5, **dadurch gekennzeichnet, dass** die Kneteraußenwand mittels eines Doppelmantels begleitbeheizt wird.

7.  Verfahren gemäß Anspruch 6, **dadurch gekennzeichnet, dass** die Kneteraußenwand mittels Heizdampf begleitbeheizt wird.

8. Verfahren gemäß Anspruch 7, **dadurch gekennzeichnet, dass** der Heizdampf einen Druck von 1,4 bis 16 bar aufweist.

9. Verfahren gemäß Anspruch 8, **dadurch gekennzeichnet, dass** die maximale Temperatur der Reaktionsmischung mindestens 105°C beträgt.

10. Verfahren gemäß einem der Ansprüche 1 bis 9, **dadurch gekennzeichnet, dass** Monomer a) zu mindestens 50 mol-% teilweise neutralisierte Acrylsäure ist.

11. Verfahren gemäß einem der Ansprüche 1 bis 10, **dadurch gekennzeichnet, dass** das Monomer a) zu 25 bis 85 mol-% neutralisiert ist.

12. Verfahren gemäß einem der Ansprüche 1 bis 11, **dadurch gekennzeichnet, dass** die Monomerlösung von 0,05 bis 1,5 Gew.-% Vernetzer b), bezogen auf das unneutralisierte Monomer a), enthält.

13. Verfahren gemäß einem der Ansprüche 1 bis 12, **dadurch gekennzeichnet, dass** die wasserabsorbierenden Polymerpartikel so klassiert werden, dass mindestens 90 Gew.-% der Partikel eine Partikelgröße von größer 150 bis 850 $\mu$m aufweisen.

14. Verfahren gemäß einem der Ansprüche 1 bis 13, **dadurch gekennzeichnet, dass** die wasserabsorbierenden Polymerpartikel zusätzlich oberflächennachvernetzt werden.

**Claims**

1. A process for producing water-absorbing polymer particles by polymerizing a monomer solution or suspension comprising

    a) an ethylenically unsaturated monomer which bears acid groups and may be at least partly neutralized,
    b) at least one crosslinker,
    c) at least one initiator,
    d) optionally one or more ethylenically unsaturated monomers copolymerizable with the monomers specified under a) and
    e) optionally one or more water-soluble polymers,

   in a kneader with a reaction volume of at least 0.1 m$^3$ and at least two shafts in axially parallel rotation, several kneading and transport elements present on the shafts bringing about conveying of the reaction mixture added at the start of the kneader in axial direction to the end of the kneader, wherein the monomer solution comprises from 0.01 to 0.2% by weight of initiator c), based on unneutralized monomer a), the outer kneader wall is trace-heated in the region of the feed and the temperature of the trace heating is at least 110°C.

2. The process according to claim 1, wherein the initiator c) is a peroxide to an extent of at least 50% by weight.

3. The process according to claim 1 or 2, wherein the initiator c) is a sodium peroxodisulfate to an extent of at least 50% by weight.

4. The process according to any of claims 1 to 3, wherein the front 50% of the outer kneader wall is trace-heated.

5. The process according to any of claims 1 to 4, wherein the entire outer kneader wall is trace-heated.

6. The process according to any of claims 1 to 5, wherein the outer kneader wall is trace-heated by means of a jacket.

7. The process according to claim 6, wherein the outer kneader wall is trace-heated by means of steam.

8. The process according to claim 7, wherein the steam has a pressure of 1.4 to 16 bar.

9. The process according to claim 8, wherein the maximum temperature of the reaction mixture is at least 105°C.

10. The process according to any one of claims 1 to 9, wherein monomer a) is acrylic acid partly neutralized to an extent of at least 50 mol%.

11. The process according to any one of claims 1 to 10, wherein monomer a) has been neutralized to an extent of 25 to 85 mol%.

12. The process according to any of claims 1 to 11, wherein the monomer solution comprises from 0.05 to 1.5% by weight of crosslinker b), based on the unneutralized monomer a).

13. The process according to any of claims 1 to 12, wherein the water-absorbing polymer particles are classified such that at least 90% by weight of the particles have a particle size of greater than 150 to 850 $\mu$m.

14. The process according to any of claims 1 to 13, wherein the water-absorbing polymer particles are additionally surface postcrosslinked.

**Revendications**

1. Procédé de fabrication de particules polymères absorbant l'eau par polymérisation d'une solution ou suspension de monomères, contenant

   a) un monomère éthyléniquement insaturé portant des groupes acides, qui peut être au moins partiellement neutralisé,
   b) au moins un agent de réticulation,
   c) au moins un initiateur,
   d) éventuellement un ou plusieurs monomères éthyléniquement insaturés, copolymérisables avec les monomères cités en a), et
   e) éventuellement un ou plusieurs polymères solubles dans l'eau,

   dans un malaxeur d'un volume réactionnel d'au moins 0,1 m$^3$ et comprenant au moins deux arbres rotatifs d'axes parallèles, plusieurs éléments de malaxage et de transport se trouvant sur les arbres, qui réalisent un transport du mélange réactionnel introduit au début du malaxeur dans la direction axiale vers la fin du malaxeur, **caractérisé en ce que** la solution de monomères contient de 0,01 à 0,2 % en poids d'initiateur c), par rapport au monomère non neutralisé a), la paroi extérieure du malaxeur dans la zone de l'entrée est chauffée par traçage et la température du chauffage par traçage est d'au moins 110 °C.

2. Procédé selon la revendication 1, **caractérisé en ce que** l'initiateur c) est un peroxyde à hauteur d'au moins 50 % en poids.

3. Procédé selon la revendication 1 ou 2, **caractérisé en ce que** l'initiateur c) est le peroxodisulfate de sodium à hauteur d'au moins 50 % en poids.

4. Procédé selon l'une quelconque des revendications 1 à 3, **caractérisé en ce que** les premiers 50 % de la paroi extérieure du malaxeur sont chauffés par traçage.

5. Procédé selon l'une quelconque des revendications 1 à 4, **caractérisé en ce que** l'ensemble de la paroi extérieure du malaxeur est chauffée par traçage.

6. Procédé selon l'une quelconque des revendications 1 à 5, **caractérisé en ce que** la paroi extérieure du malaxeur est chauffée par traçage au moyen d'une double enveloppe.

7. Procédé selon la revendication 6, **caractérisé en ce que** la paroi extérieure du malaxeur est chauffée par traçage au moyen de vapeur surchauffée.

8. Procédé selon la revendication 7, **caractérisé en ce que** la vapeur surchauffée présente une pression de 1,4 à 16 bar.

9. Procédé selon la revendication 8, **caractérisé en ce que** la température maximale du mélange réactionnel est d'au moins 105 °C.

**10.** Procédé selon l'une quelconque des revendications 1 à 9, **caractérisé en ce que** le monomère a) est l'acide acrylique partiellement neutralisé à hauteur d'au moins 50 % en moles.

**11.** Procédé selon l'une quelconque des revendications 1 à 10, **caractérisé en ce que** le monomère a) est neutralisé à hauteur de 25 à 85 % en moles.

**12.** Procédé selon l'une quelconque des revendications 1 à 11, **caractérisé en ce que** la solution de monomères contient de 0,05 à 1,5 % en poids d'agent de réticulation b), par rapport au monomère a) non neutralisé.

**13.** Procédé selon l'une quelconque des revendications 1 à 12, **caractérisé en ce que** les particules polymères absorbant l'eau sont classifiées de sorte qu'au moins 90 % en poids des particules présentent une taille de particule de plus de 150 à 850 $\mu$m.

**14.** Procédé selon l'une quelconque des revendications 1 à 13, **caractérisé en ce que** les particules polymères absorbant l'eau sont en outre post-réticulées en surface.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 01038402 A1
- WO 03022896 A1
- WO 2006034806 A1
- CH 664704 A5
- EP 0517068 A1
- WO 9712666 A1
- DE 2123956 A1
- EP 0603525 A1
- DE 19536944 A1
- DE 4118884 A1
- WO 02055469 A1
- WO 03078378 A1
- WO 2004035514 A1
- EP 0530438 A1
- EP 0547847 A1
- EP 0559476 A1
- EP 0632068 A1
- WO 9321237 A1
- WO 03104299 A1
- WO 03104300 A1
- WO 03104301 A1
- DE 10331450 A1
- DE 10331456 A1
- DE 10355401 A1
- DE 19543368 A1
- DE 19646484 A1
- WO 9015830 A1
- WO 02032962 A2
- EP 0083022 A2
- EP 0543303 A1
- EP 0937736 A2
- DE 3314019 A1
- DE 3523617 A1
- EP 0450922 A2
- DE 10204938 A1
- US 6239230 B
- DE 4020780 C1
- DE 19807502 A1
- DE 19807992 C1
- DE 19854573 A1
- DE 19854574 A1
- DE 10204937 A1
- DE 10334584 A1
- EP 1199327 A2
- WO 03031482 A1
- DE 3713601 A1
- EP 0640330 A1

**In der Beschreibung aufgeführte Nicht-Patentliteratur**

- Modern Superabsorbent Polymer Technology. **F.L. BUCHHOLZ ; A.T. GRAHAM.** Monographie. Wiley-VCH, 1998, 71-103
- Standard Test Methods for the Nonwovens Industry. 2005